# EUROPEAN PATENT APPLICATION

(11) **EP 4 703 992 A1**
(43) Date of publication of application: **04.03.2026**
(21) Application number: 23935415.2
(22) Date of filing: 28.04.2023
(51) Int. Cl.: G06Q 10/0637, G06Q 50/26

(54) **MEASURE EVALUATION METHOD, INFORMATION PROCESSING DEVICE, AND MEASURE EVALUATION PROGRAM**

(71) Applicant: FUJITSU LIMITED, Kawasaki-shi, Kanagawa 211-8588 (JP)
(72) Inventor: AMEMIYA, Satoshi, Kawasaki-shi, Kanagawa 211-8588 (JP); MIZOUCHI, Tsuyoshi, Kawasaki-shi, Kanagawa 211-8588 (JP); INOMATA, Akihiro, Kawasaki-shi, Kanagawa 211-8588 (JP)
(74) Representative: Hoffmann Eitle
(86) International application number: PCT/JP2023/016965
(87) International publication number: WO 2024/224640

(57) **Abstract**

A policy evaluation method executed by a computer, includes, when acquiring a first policy that is a policy including a conditional branch and a node connected by a directed edge, referring to a storage unit that stores a plurality of second policies and an evaluation value of each of the plurality of second policy, the evaluation value indicating the number of targets to which a plurality of targets is assigned to a node by the policy, and calculating a similarity measure between the first policy and each of the plurality of second policies, and outputting an evaluation value of a similar policy that is a policy including the conditional branch and the node having the similarity measure equal to or greater than a threshold among the plurality of second policies.

## Description

### Technical Field

The present invention relates to a policy evaluation method, an information processing apparatus, and a policy evaluation program.

### Background Art

As one of the workflows, there is known a policy flow graph in which a flow of allocating an object to be a target of policies in various fields such as medical care, nursing care, and administration, for example, a human to a service or the like for achieving the purpose of the policy is schematized.

As one of the techniques for supporting the planning of such a policy, the following insurance medical data analysis system has been proposed. For example, in the insurance medical data analysis system, vector information is calculated from national (first group) health and medical care (healthcare) data, and model information (prediction model) is generated from this vector information and vector information of policies implemented in a specific region (second group) and healthcare data for policy. By applying the healthcare data of another region (third group) to this model information, it becomes possible to predict an effect in a case where a policy implemented in a specific region (second group) is implemented in another region (third group), and it becomes possible to support creation of a new policy for healthcare (medical care, health checkup, or nursing) .

### Citation List

### Patent Literature

Patent Document 1: Japanese Laid-open Patent Publication No. 2021-89523

### Summary of invention

### Technical Problem

However, the effect of the policy predicted by the above-described insurance medical data analysis system is merely a prediction value using model information and does not reach the evaluation based on the actual value, and thus there is a side in which it is difficult to improve the evaluation accuracy of the inexperienced policy.

In one aspect, an object of the present invention is to provide a policy evaluation method, an information processing apparatus, and a policy evaluation program capable of improving evaluation accuracy of an inexperienced policy.

### Solution to Problem

According to an aspect of the embodiment of the invention, a policy evaluation method executed by a computer, the policy evaluation method includes: when acquiring a first policy that is a policy including a conditional branch and a node connected by a directed edge, referring to a storage unit that stores a plurality of second policies and an evaluation value of each of the plurality of second policies, the evaluation value indicating a number of targets to which a plurality of targets is assigned to a node by the policy, and calculating a similarity measure between the first policy and each of the plurality of second policies; and outputting an evaluation value of a similar policy that is a policy including the conditional branch and the node having the similarity measure equal to or greater than a threshold among the plurality of second policies. Advantageous Effects of Invention

According to one embodiment, it is possible to improve the evaluation accuracy of an inexperienced policy.

### Brief Description of Drawings

FIG. 1 is a block diagram illustrating a functional configuration example of a server apparatus.
FIG. 2 is a diagram illustrating a policy flow graph.
FIG. 3 is a diagram illustrating a specific example of the policy flow graph.
FIG. 4 is a diagram illustrating an example of policy information.
FIG. 5 is a schematic diagram illustrating an example of generating a feature vector.
FIG. 6 is a schematic diagram illustrating an example of extracting features.
FIG. 7 is a diagram illustrating an example of similar or dissimilar existing policies.
FIG. 8 is a diagram illustrating an example of similar or dissimilar existing policies.
FIG. 9 is a diagram illustrating an example of an evaluation value graph.
FIG. 10 is a diagram illustrating a display example of a client terminal.
FIG. 11 is a flowchart illustrating a procedure of an output process of a similar policy.
FIG. 12 is a schematic diagram illustrating an example of a machine learning model.
FIG. 13 is a flowchart illustrating a procedure of an output process of a similar policy.
FIG. 14 is a diagram illustrating an exemplary hardware configuration.

### Description of Embodiments

Hereinafter, modes for implementing a policy evaluation method, an information processing apparatus, and a policy evaluation program according to the present application (hereinafter, described as "embodiment") will be described with reference to the accompanying drawings. Each embodiment merely illustrates an example and a side surface, and numerical values, function ranges, use scenes, and the like are not limited by such an example. Then, the embodiments can be adaptively combined within a range in which the process contents do not contradict each other.

### <First Embodiment>

### <System Configuration>

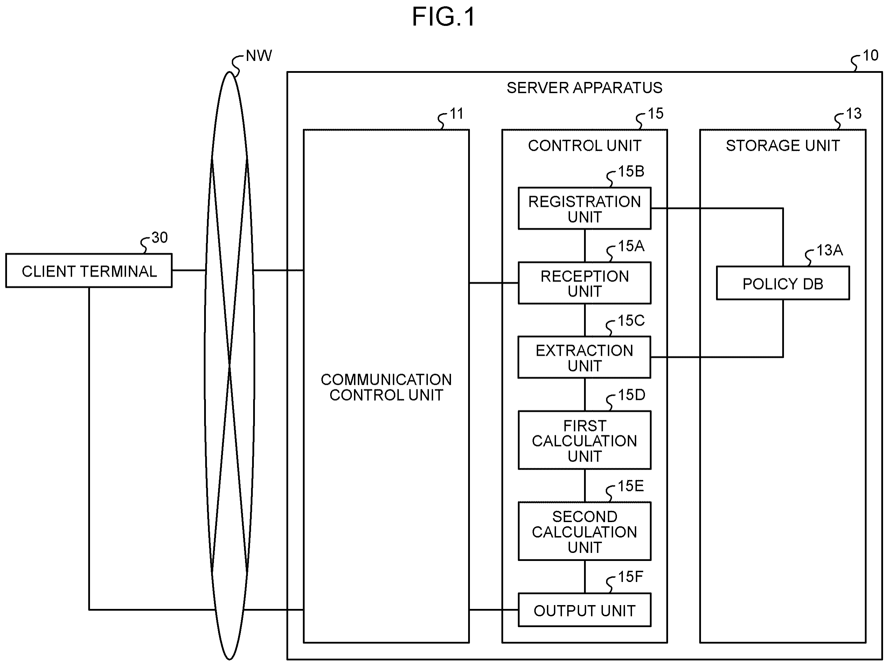

FIG. 1 is a block diagram illustrating a functional configuration example of a server apparatus 10. The server apparatus 10 illustrated in FIG. 1 provides a platform as a data platform capable of sharing, cross-referencing, and updating policy flow data.

For example, the server apparatus 10 can provide the function of a platform of the above-described data platform as a cloud service by executing platform as a service (PaaS) type middleware or software as a service (SaaS) type application.

As illustrated in FIG. 1, the server apparatus 10 can be communicably connected to a client terminal 30 via a network NW. For example, the network NW may be any type of communication network such as the Internet or a local area network (LAN) regardless of whether it is wired or wireless. Note that FIG. 1 illustrates an example in which one client terminal 30 is connected to one server apparatus 10, but any number of client terminals 30 may be connected.

The client terminal 30 is a terminal device that receives the provision of the data platform. For example, the client terminal 30 can be used by a policy planner as an example of an entity involved in implementing the policy, for example, related parties such as a local government. Note that, as an example, the client terminal 30 may be realized by any computer such as a personal computer, a smartphone, a tablet terminal, or a wearable terminal.

### <Policy Flow Graph>

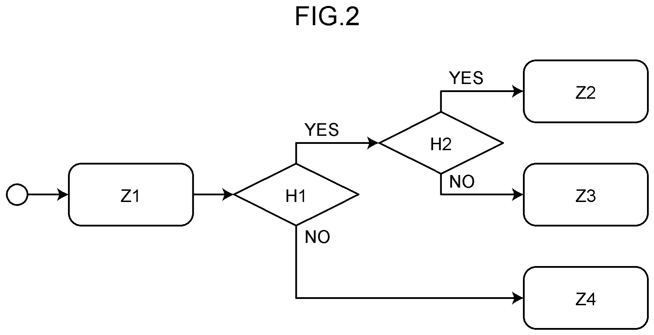

A flow graph for the policy described above is illustrated in FIG. 2. FIG. 2 is a diagram illustrating the policy flow graph. Z1, Z2, Z3, and Z4 illustrated in FIG. 2 indicate, for example, services provided by the administrator to the user. In addition, these components may be referred to as "service implementation components". Specific examples of the service include, for example, in the medical field, "intervention" to which an object, for example, a resident or the like, to be a target of the policy such as undergoing a medical examination or an examination by a medical specialist, is assigned, and "no intervention" such as follow-up, but are not limited to the policies in the medical field.

H1 and H2 indicate, for example, conditional branches including conditions. In addition, these components may be referred to as "conditional branch components". Specific examples of the condition include, for example, in the medical field, an estimated glemerular filtration rate (eGFR) is less than the threshold value, a hemoglobin A1c value (HbA1c) is less than the threshold value, and a urinary protein value is equal to or more than the threshold value, but are not limited to the conditions in the medical field.

Z1, Z2, Z3, and Z4, and H1 and H2 may be referred to as a "component", respectively. Such a "component" can correspond to an example of a "node" in terms of graph data. Furthermore, the connection between nodes can correspond to an example of an "edge" including an "directed edge" and the like.

Note that, in the present embodiment, planning of a policy in the medical field will be described as an example, but the present invention is not limited thereto. The above-described embodiment may be used for planning various policies such as work having conditional branches, tests, and questionnaires. Also in these cases, the same operation and effect as those of the above-described embodiment can be obtained.

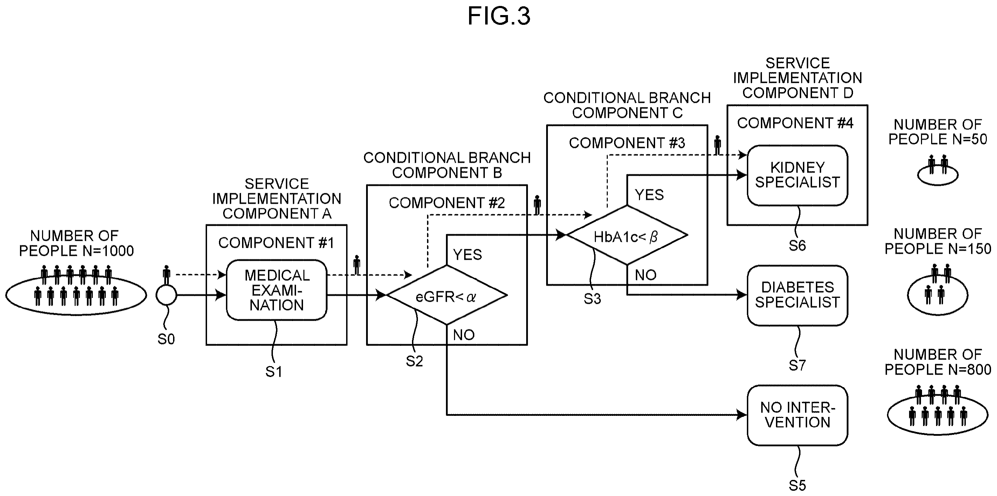

FIG. 3 is a diagram illustrating a specific example of the policy flow graph. As illustrated in FIG. 3, a policy is modeled as a workflow including a combination of components such as conditional branching and service implementation. Then, the number of people who receive each service is output from the model trained by accumulating information and parameters of the flow of people from the actual value when used for each conditional branch component. As a result, the local government can incorporate policies suitable for the local government among the policies implemented by other local governments in consideration of the resources of the local government.

In the example illustrated in FIG. 3, the number of people N = 1000 is input in a reference sign S0. In a reference sign S1, a component #1 as a service implementation component A is set to "medical examination". In a reference sign S2, a component #2 as a conditional branch component B is set to "eGFR < α". In a case where "eGFR < α" is not satisfied (see NO route of reference sign S2), it is determined that "no intervention" in the citizen by the specialist as indicated by a reference sign S5.

On the other hand, in a case where "eGFR < α" is satisfied (see YES route of reference sign S2), the component #3 as the conditional branch component C is set to "HbA1c < β" as indicated by a reference sign S3. In a case where "HbA1c < β" is satisfied (see YES route of the reference sign S3), as indicated by a reference sign S6, the component #4 as the conditional branch component D is set as a "kidney specialist", and it is determined that the intervention of "kidney specialist" is necessary for the citizen. On the other hand, in a case where "HbA1c < β" is not satisfied (see NO route of the reference sign S3), as indicated by a reference sign S7, it is determined that the intervention of the "diabetes specialist" is necessary for the citizen.

In the example illustrated in FIG. 3, as indicated by arrows, the number of people flowing through the components #1, #2, #3, and #4 in this order is predicted. For example, in the policy flow graph illustrated in FIG. 3, the result of allocating the number of people N = 1000 to the interventions Z2 to Z4 is as follows. 50 people are assigned to the intervention Z2. 150 people are assigned to the intervention Z3. Furthermore, 800 people are assigned to the intervention Z4.

Here, another specific example of use of the policy flow graph will be described with reference to FIGS. 2 and 3. The server apparatus 10 searches the policy flow graph using the attribute information of the person of the organization to which the policy planner belongs. The server apparatus 10 specifies a node into which a person is classified among nodes located at the end constituting the policy flow graph. As a result, the local government to which the policy has been applied can specify the medical institution to be recommended to the person in consideration of the health condition of the person belonging to the local government and the resource of the local government.

First, the server apparatus 10 specifies a person included in the organization to which the policy planner belongs. For example, the server apparatus 10 specifies a resident of the local government. Next, the server apparatus 10 searches the output policy flow graph using the attribute information of the person of the organization to which the policy planner belongs, thereby specifying a node in which the specified person is classified among the nodes located at the end constituting the policy flow graph. The attribute information is biological information specified by analyzing a body fluid of the person. The attribute information is an estimated glomerular filtration rate, a hemoglobin A1c value, a urinary protein value, and the like. The body fluid includes blood, lymph fluid, tissue fluid (intertissue fluid, intercellular fluid, interstitial fluid) sweat, tears, nasal discharge, urine, semen, vaginal fluid, amniotic fluid, and milk.

At this time, the server apparatus 10 compares the attribute information of the person with the condition included in the conditional branch component to specify the node in which the person is classified. The server apparatus 10 specifies a node in which the specified person is classified among the nodes located at the end. Then, the server apparatus 10 sets a medical institution indicated by the specified node to be classified as a medical institution to be recommended to the specified person. The medical institution indicated by the node is a kidney specialist, a diabetes specialist, or the like.

Hereinafter, the policy flow graph may be abbreviated as the "policy flow". Furthermore, in the policy flow, a policy flow corresponding to a draft may be described as a "draft flow", and a policy flow corresponding to an existing policy may be described as an "existing policy flow". Note that the term "draft" as used herein refers to a policy designated as a draft at the time of planning policies. For example, one of existing policies can be designated as it is, a changed policy in which a part of the existing policies has been changed may be designated, or a newly created new policy may be designated.

### <Data Platform>

In the above data platform, a policy flow may be shared in an arbitrary framework. Merely as an example, the above-described data platform can share a policy flow among organizations in the world, for example, public organizations such as local governments.

Through the client terminal 30, the policy planner can refer to the templates of the existing policies in the world collected in the above data platform. For example, among the templates collected in the data platform, the draft can be updated by incorporating entire or a part of the existing policies similar to the draft.

As described above, at the time of planning policies, from the viewpoint of administrative (political) easiness of execution, it is important whether or not policies similar to those in the past have been taken. For this reason, the importance of comparing the flow graph of the policy as a draft with the flow graph of the existing policies as a reference is increased.

### <Configuration of Server Apparatus 10>

FIG. 1 schematically illustrates blocks related to a data platform included in the server apparatus 10. As illustrated in FIG. 1, the server apparatus 10 includes a communication control unit 11, a storage unit 13, and a control unit 15. Note that FIG. 1 merely illustrates excerpted functional units related to the above-described data platform, and functional units other than those illustrated may be provided in the server apparatus 10.

The communication control unit 11 is a functional unit that controls communication with other devices such as the client terminal 30. As an example, the communication control unit 11 can be realized by a network interface card such as a LAN card. As one aspect, the communication control unit 11 receives a registration request of policy information including a policy flow and an output request of a similar policy of a draft from the client terminal 30, or outputs a similar policy of the draft to the client terminal 30.

The storage unit 13 is a functional unit that stores various data. As an example, the storage unit 13 is realized by an internal, external, or auxiliary storage of the server apparatus 10. For example, the storage unit 13 stores a policy database (DB) 13A. Note that the policy DB will be described together in a scene where reference, generation, or registration is executed.

The control unit 15 is a functional unit that performs overall control of the server apparatus 10. For example, the control unit 15 can be realized by a hardware processor. In addition, the control unit 15 may be realized by hard-wired logic. As illustrated in FIG. 1, the control unit 15 includes a reception unit 15A, a registration unit 15B, an extraction unit 15C, a first calculation unit 15D, a second calculation unit 15E, and an output unit 15F.

The reception unit 15A is a processing unit that receives various requests from the client terminal 30. As one aspect, the reception unit 15A can receive a registration request of policy information including a policy flow from the client terminal 30. As another aspect, the reception unit 15A can receive an output request of a similar policy of a draft.

The registration unit 15B is a processing unit that registers the policy information in the policy DB 13A of the storage unit 13. As an example, if the registration request of the policy information is received by the reception unit 15A, the registration unit 15B registers the policy information in the policy DB 13A.

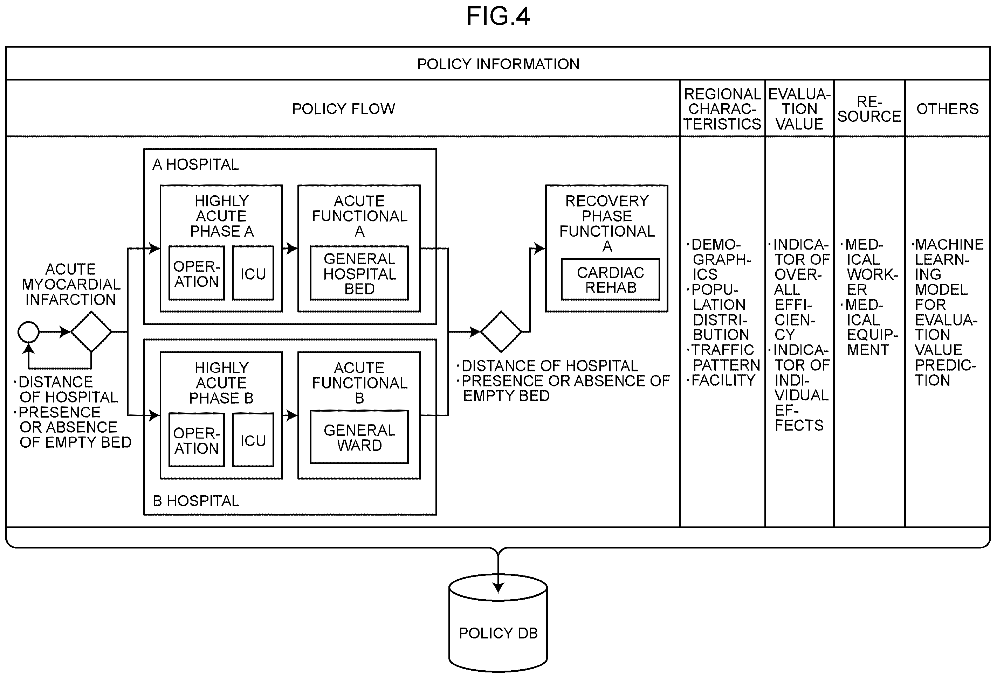

FIG. 4 is a diagram illustrating an example of the policy information. As illustrated in FIG. 4, the policy information received in the registration request may include a policy flow of an existing policy, an organization to which a policy planner of the policy flow belongs, for example, regional characteristics corresponding to a local government, an evaluation value of the policy flow, resources of the organization, and other information. For example, the regional characteristics may include regional demographics, regional population distribution, regional traffic patterns, facilities deployed in the regions, and the like. In addition, the evaluation value may include an actual value of an indicator of overall efficiency, an actual value of an indicator of individual effects, and the like. Further, the resources may include a size, a distribution, or the like related to a quantity of medical workers, medical equipment, or the like. In addition, resources required at the time of implementing the policy flow may be further included. The other pieces of information may include a machine learning model that outputs an evaluation value using a policy flow, regional characteristics, and the like as inputs. A set of policy information of such existing policies is managed by the facility DB.

The extraction unit 15C is a processing unit that extracts feature amounts regarding a policy flow and regional characteristics. As an example, the extraction unit 15C can start the process in a case where the reception unit 15A receives an output request of the similar policy of the draft. A draft α designated in the output request of such a similar policy may receive designation from existing policies already registered in the policy DB 13A, or may receive designation of a policy not registered in the policy DB 13A from the client terminal 30.

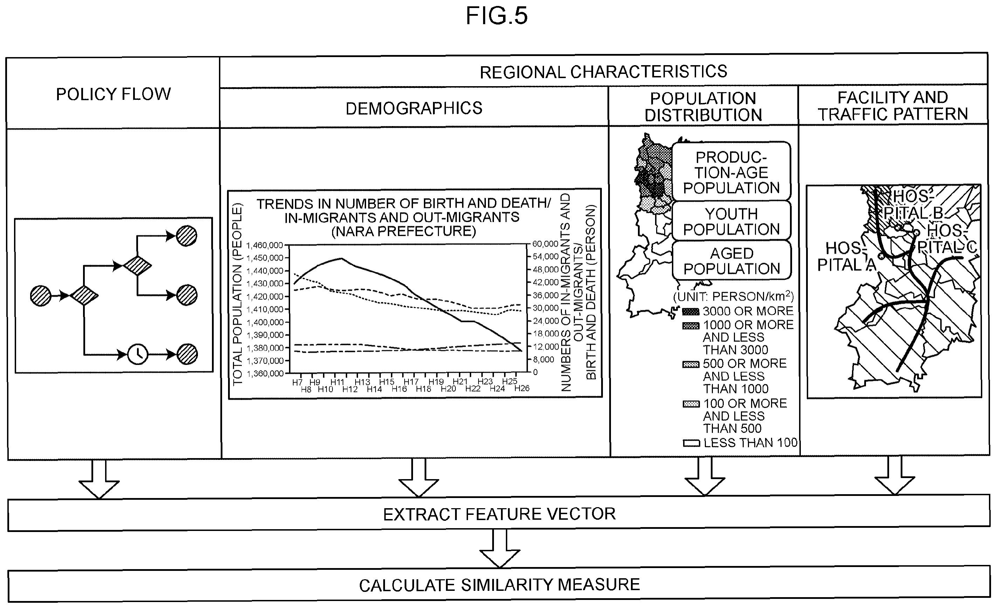

For example, the extraction unit 15C extracts feature amounts of a policy flow and regional characteristics for each of a draft designated by the output request of the similar policy described above and the existing policies stored in the policy DB 13A. FIG. 5 is a schematic diagram illustrating an example of generating a feature vector. FIG. 5 illustrates demographics, population distributions, facilities, traffic patterns, and the like as examples of the regional characteristics. As illustrated in FIG. 5, the extraction unit 15C extracts a feature vector in which a feature amount obtained by quantifying the features of the policy flow and a feature amount obtained by quantifying the features of the demographics, a feature amount obtained by quantifying the features of the population distribution, and a feature amount obtained by quantifying the features of the facilities and the traffic patterns are expressed in a vector format.

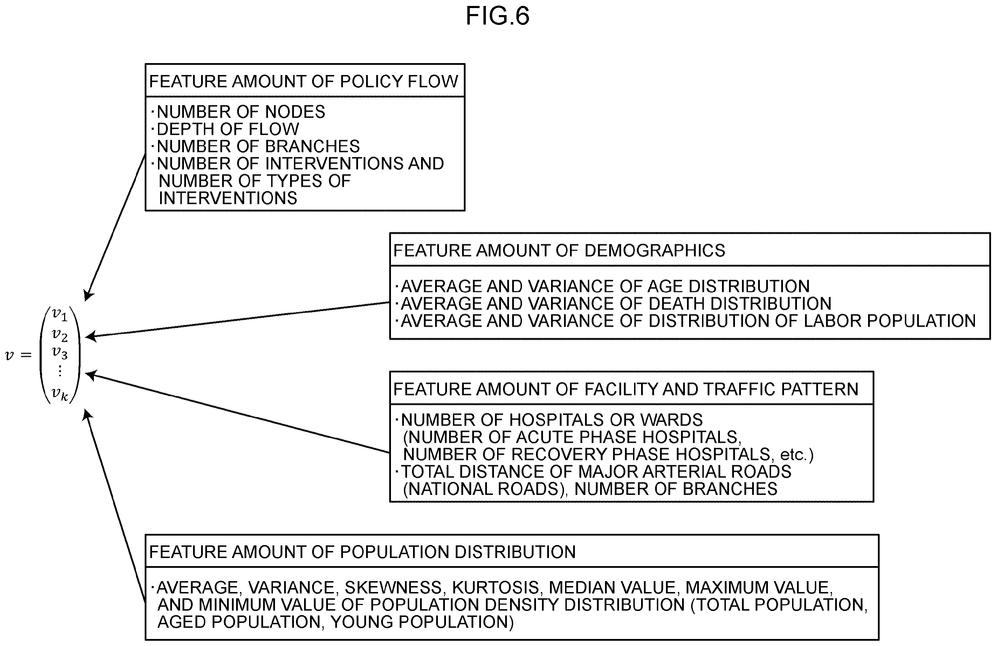

More specifically, the extraction unit 15C can generate a feature vector by executing the feature extraction illustrated in FIG. 6 from the policy flow and the regional characteristics. FIG. 6 is a schematic diagram illustrating an example of extracting features. As illustrated in FIG. 6, the extraction unit 15C can extract the number of nodes, the depth of the flow, the number of branches, the number of interventions, the number of types of interventions, and the like as the feature amount of the policy flow. In addition, the extraction unit 15C can extract statistical values such as the average and variance of the age distribution, statistical values such as the average and variance of the death distribution, and the like as the feature amount of the demographics. Furthermore, the extraction unit 15C can extract the number of hospitals, the number of wards, or the like for each medical function such as the acute phase or the recovery phase as the feature amount of the facility. In addition, the extraction unit 15C can extract, as the feature amount of the population distribution, a statistical value of the population density distribution such as the total population, the aged population, the young population, and the execution of the production age, for example, an average, a variance, a skewness, a kurtosis, a median value, a maximum value, a minimum value, and the like.

Returning to the description of FIG. 1, the first calculation unit 15D is a processing unit that calculates a similarity measure between a draft and an existing policies. As an example, the first calculation unit 15D calculates the similarity measure between the feature vector extracted from the draft by the extraction unit 15C and the feature vector extracted for each of the N existing policies by the extraction unit 15C, for example, an inner product or a cosine similarity. Note that the similarity measure may be calculated by normalizing the distance calculated between the feature vectors of the draft and the existing policies.

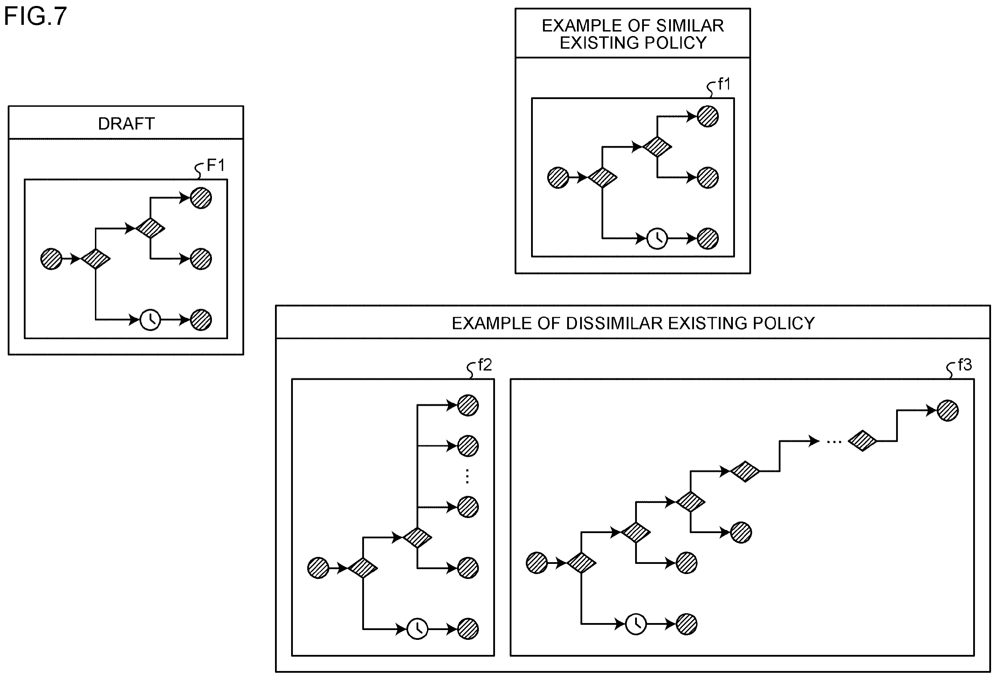

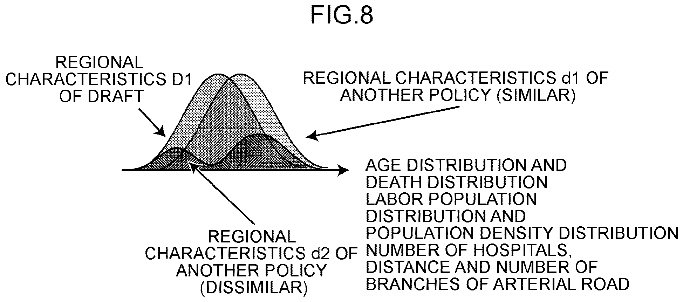

FIGS. 7 and 8 are diagrams illustrating an example of similar or dissimilar existing policies. FIG. 7 illustrates an existing policy f1 having a graph structure similar to that of a draft policy flow F1, and existing policies f2 and f3 having a graph structure dissimilar to that of the draft policy flow F1. FIG. 8 illustrates an existing policy having a regional characteristic d1 similar to the regional characteristic D1 of the draft and an existing policy having a regional characteristic d2 dissimilar to the regional characteristic D1 of the draft. Note that the regional characteristics D1, d1, and d2 illustrated in FIG. 8 may include the age distribution, the death distribution, the labor population distribution, the population density distribution, the number of hospitals, the total distance of the arterial road, the number of branches of the arterial road, and the like illustrated in FIG. 6.

As illustrated in FIG. 7, in the existing policy f1 having a graph structure similar to that of the policy flow F1 of the draft, the distance between the feature amounts of the policy flows becomes short, and as a result of increasing the similarity measure with respect to the policy flow F1 of the draft, the similar policy is likely to be implemented. On the other hand, in the existing policies f2 and f3 having a graph structure dissimilar to that of the policy flow F1 of the draft, the distance between the feature amounts of the policy flows becomes long, and as a result of decreasing the similarity measure with respect to the policy flow F1 of the draft, the dissimilar policy is likely to be implemented.

In addition, as illustrated in FIG. 8, in the existing policies having the regional characteristics d1 similar to the regional characteristics D1 of the draft, the distance between the feature amounts of the regional characteristics becomes short, and as a result of increasing the similarity measure to the draft, the similar policies are likely to be taken. On the other hand, in the existing policies having the regional characteristics d2 dissimilar to the regional characteristics D1 of the draft, the distance between the feature amounts of the regional characteristics becomes long, and as a result of decreasing the similarity measure to the draft, the dissimilar policies are likely to be taken.

Returning to the description of FIG. 1, the second calculation unit 15E is a processing unit that calculates an interval of the evaluation value of the similar policy of the draft. As an example, the second calculation unit 15E extracts, as a similar policy, an existing policy having a similarity measure calculated by the first calculation unit 15D equal to or greater than a threshold among the N existing policies included in the policy DB 13A. Subsequently, the second calculation unit 15E refers to evaluation values of M (≤ N) similar policies of which the similarity measure is equal to or greater than the threshold. Under the situation where the evaluation values of the M similar policies are referred to in this manner, the second calculation unit 15E can calculate intervals with the maximum and minimum evaluation values and calculate the confidence interval corresponding to the similarity measure distribution of the evaluation values.

As one aspect, the second calculation unit 15E calculates the maximum evaluation value and the minimum evaluation value corresponding to the evaluation axis among the evaluation values of the M similar policies for each of the K evaluation axes. The intervals with the maximum and minimum evaluation values in the K-dimensional evaluation space can be defined by the maximum evaluation value and the minimum evaluation value calculated for each of the K evaluation axes in this manner.

As another aspect, the second calculation unit 15E calculates a confidence interval of M similar policies, for example, a 90% confidence interval or a 95% confidence interval, on the basis of the distribution of the similarity measure of the M similar policies for each of the K evaluation axes. As a result, it is possible to set a confidence interval in which an outlier or an abnormal value of the evaluation value is excluded on the K-dimensional evaluation space.

The output unit 15F is a processing unit that outputs various types of information to the client terminal 30. As one aspect, the output unit 15F can output, to the client terminal 30, evaluation values of M similar policies of which similarity measure calculated by the first calculation unit 15D is equal to or greater than a threshold among the N existing policies included in the policy DB 13A. At this time, the output unit 15F can generate an evaluation value graph by plotting the evaluation values of the M similar policies corresponding to the evaluation axes for each evaluation axis. Furthermore, the output unit 15F can plot intervals with the maximum and minimum evaluation values calculated by the second calculation unit 15E on the evaluation value graph, or plot the confidence interval of the M similar policies calculated by the second calculation unit 15E.

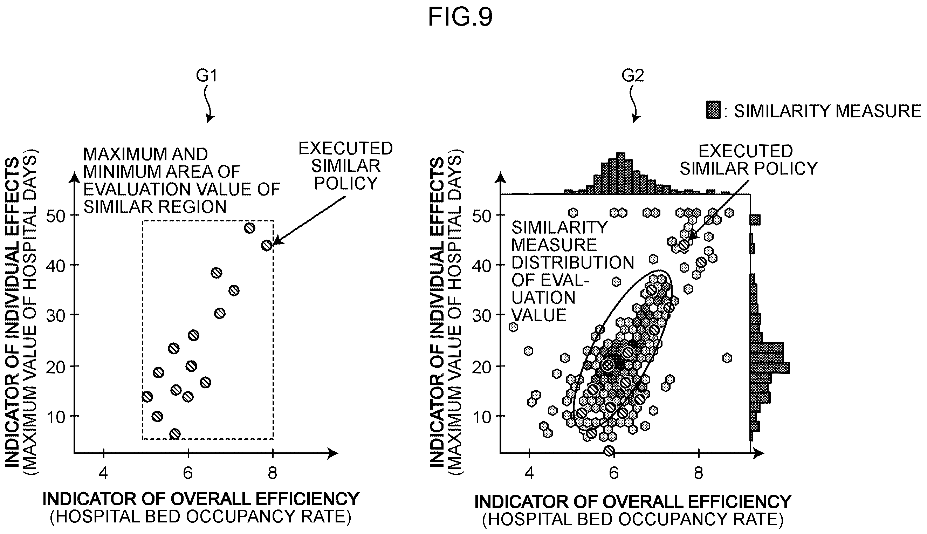

FIG. 9 is a diagram illustrating an example of an evaluation value graph. FIG. 9 illustrates an indicator of overall efficiency (hospital bed occupancy rate) and an indicator of individual effects (maximum value of hospital days) as examples of evaluation axes. As illustrated in FIG. 9, in an evaluation value graph G1 and an evaluation value graph G2, evaluation values (actual values) of M similar policies corresponding to the evaluation axes are plotted for each of the two evaluation axes. Among them, in the evaluation value graph G1, the intervals with the maximum and minimum evaluation values, that is, a broken line frame is plotted. On the other hand, in the evaluation value graph G2, the similarity measure of the M similar policies is further plotted in association with the evaluation values of the M similar policies for each of the two evaluation axes, and a confidence interval of the M similar policies, that is, an elliptical area is plotted. According to the evaluation value graph G1 and the evaluation value graph G2, since an inexperienced policy can be evaluated on the basis of the actual values, it is possible to improve the evaluation accuracy of the inexperienced policy.

As another aspect, the output unit 15F can output a list of M similar policies in which the similarity measure calculated by the first calculation unit 15D is equal to or greater than a threshold. Furthermore, the output unit 15F can narrow down and output similar policies in which the evaluation value satisfies a predetermined condition among the M similar policies, for example, similar policies corresponding to the best value or the highest specific number, and output similar policies in which the evaluation value satisfies a predetermined condition as proposed policies. In addition, the output unit 15F can output M similar policies or similar policies in which the resource required at the time of implementation satisfies a predetermined condition among similar policies in which the evaluation value satisfies a predetermined condition, for example, similar policies within an allowable range set on the basis of the resource of the local government of the policy planner.

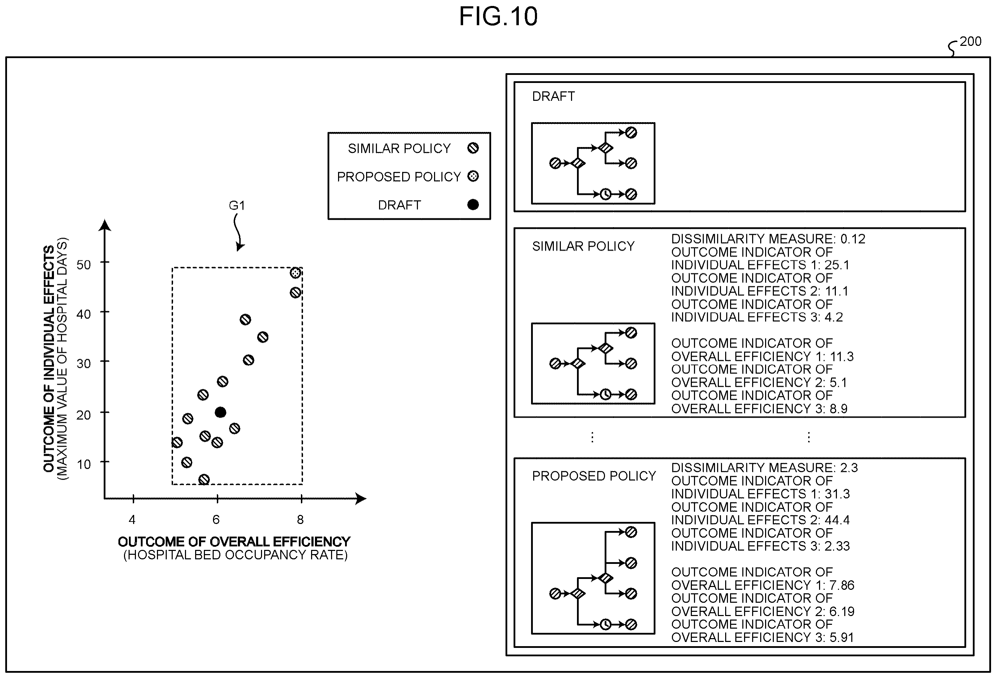

FIG. 10 is a diagram illustrating a display example of the client terminal 30. As illustrated in FIG. 10, the evaluation value graph G1 illustrated in FIG. 9 is displayed in a window 200. Furthermore, in the window 200, a policy flow of a draft is displayed, and a policy flow of M similar policies whose similarity measure is equal to or greater than a threshold and a list of evaluation values for each evaluation axis of the M similar policies are displayed. Furthermore, in the window 200, a similar policy having the best evaluation value among the M similar policies is displayed as a proposed policy. According to such a window 200, it is possible to output an existing policy having regional characteristics similar to the regional characteristics of the draft as a similar policy.

Note that although FIG. 10 illustrates an example in which a list of M similar policies is displayed, it is also possible to display a policy flow of a similar policy for which user designation has been received from the evaluation value graph G1 or the evaluation value graph G2. In addition, it is also possible to display a similar policy having the minimum evaluation value in each evaluation axis among intervals with the maximum and minimum evaluation values in the evaluation value graph G1, or a similar policy having the maximum evaluation value in each evaluation axis. Furthermore, a policy flow of similar policies located at the shortest position from the center or the center of gravity of the similarity measure distribution included in the evaluation value graph G2 may be displayed.

### <Procedure of Process>

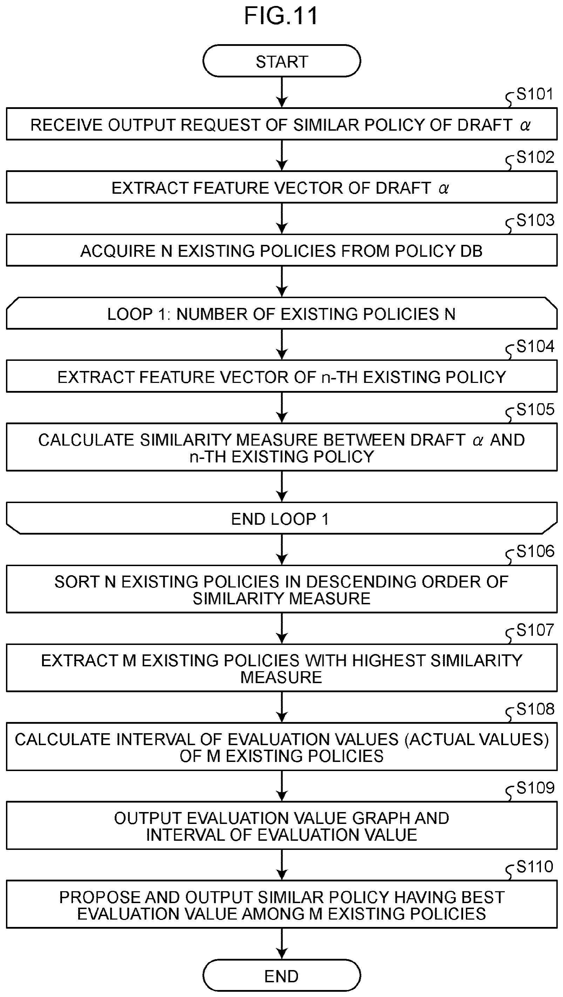

FIG. 11 is a flowchart illustrating a procedure of an output process of the similar policies. As an example, this process can be started in a case where the reception unit 15A receives an output request of the similar policy of the draft.

As illustrated in FIG. 11, when an output request of the similar policy of the draft α is received by the reception unit 15A (step S101), the extraction unit 15C executes the following process. That is, the extraction unit 15C extracts a feature vector in which a feature amount obtained by quantifying the features of the policy flow of the draft α and a feature amount obtained by quantifying the features of the regional characteristics of the draft α are expressed in a vector format (step S102). Subsequently, the extraction unit 15C acquires N existing policies included in the policy DB 13A stored in the storage unit 13 (step S103).

Thereafter, loop processing 1 that repeats the processes of the following step S104 and the following step S105 is executed by the number of times corresponding to the number of existing policies N acquired in step S103.

That is, the extraction unit 15C extracts a feature vector in which a feature amount obtained by quantifying the features of the policy flow of the n-th existing policy and a feature amount obtained by quantifying the features of the regional characteristics of the n-th existing policy are expressed in a vector format (step S104).

Then, the first calculation unit 15D calculates similarity measure between the feature vector of the draft α extracted in step S102 and the feature vector of the n-th existing policy extracted in step S104 (step S105).

By repeating such loop processing 1, the similarity measure is calculated for each of the N existing policies.

Thereafter, the second calculation unit 15E sorts the N existing policies in descending order of the similarity measure calculated in step S105 (step S106). Subsequently, the second calculation unit 15E extracts M existing policies with the highest similarity measure (step S107).

Then, the second calculation unit 15E calculates an interval of evaluation values (actual values) of the M existing policies, for example, the maximum and minimum intervals or a confidence interval (step S108). Subsequently, the output unit 15F further plots an interval of the evaluation value calculated in step S108 on an evaluation value graph in which the evaluation values of the M similar policies corresponding to the evaluation axis are plotted for each evaluation axis, and causes the client terminal 30 to display the interval (step S109).

Furthermore, the output unit 15F causes the client terminal 30 to display, as a proposed policy, a similar policy whose evaluation value satisfies a predetermined condition among the M similar policies, for example, a similar policy corresponding to the best value or the highest specific number (step S110), and ends the process.

### <One Aspect of Effect>

As described above, the server apparatus 10 according to the present embodiment calculates the similarity measure of the flow graph between the draft and the existing policies when the flow graph of the draft is designated, and outputs the evaluation value stored in association with the similar policy having the similarity measure equal to or greater than the threshold among the flow graphs of the existing policies. Therefore, the inexperienced policy can be evaluated on the basis of actual values. Therefore, according to the server apparatus 10 according to the present embodiment, it is possible to improve the evaluation accuracy of the inexperienced policy.

Further, the server apparatus 10 according to the present embodiment calculates the similarity measure of the regional characteristics between the draft and the existing policies when the flow graph of the draft is designated, and outputs the flow graph of the similar policy having the similarity measure equal to or greater than the threshold among the flow graphs of the existing policies. Therefore, according to the server apparatus 10 according to the present embodiment, it is possible to output an existing policy having regional characteristics similar to the regional characteristics of the draft as a similar policy.

### <Second Embodiment>

Although the embodiments relating to the disclosed apparatus have been described so far, the present invention may be implemented in various different forms other than the above-described embodiments. Therefore, other embodiments included in the present invention will be described below.

### <Machine Learning Model for Evaluation Value Prediction>

The draft prepared by the policy planner is not necessarily one draft. For example, there is a use scene in which K drafts are prepared by arranging a part of one or more existing policies. In this case, as the number of the draft increases, the load of the process illustrated in FIG. 11 also increases. From such an aspect, it is possible to narrow down the number of drafts to be compared with the existing policies using a machine learning model that outputs an evaluation value using a policy flow, regional characteristics, and the like as inputs.

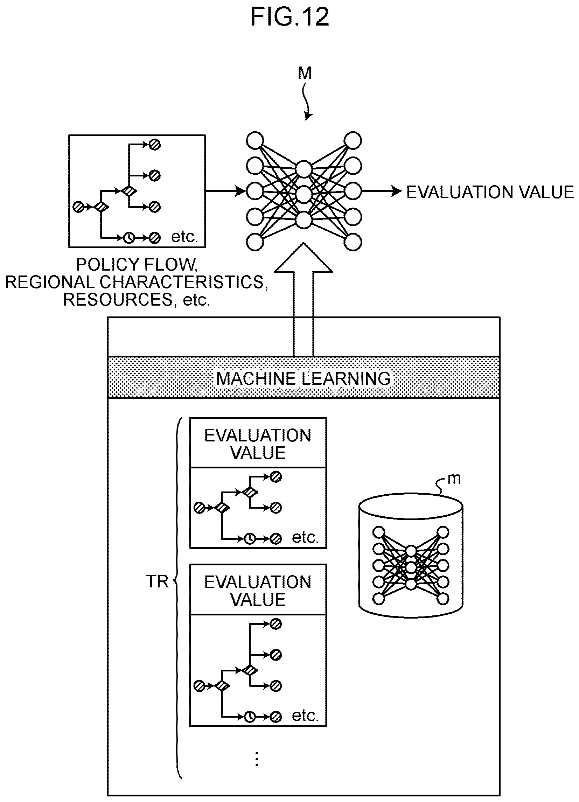

FIG. 12 is a schematic diagram illustrating an example of the machine learning model. As illustrated in FIG. 12, a machine learning model m is used to predict the evaluation values of the policy flows of the K drafts. For example, the machine learning model m may be realized by a neural network, a support vector machine, gradient boosting, or the like. For training of such a machine learning model m, a data set TR including training data associated with a correct answer label of a feature amount and an evaluation value of at least one of a policy flow, regional characteristics, and a resource can be used.

For example, in the training phase, the machine learning model m can be trained according to an arbitrary machine learning algorithm, for example, deep learning, with the feature amount of at least one of the policy flow, the regional characteristics, and the resource as an explanatory variable of the machine learning model m and the label as an objective variable of the machine learning model m. As a result, a trained machine learning model M is obtained.

In the prediction phase, the feature amount of at least one of the policy flow, the regional characteristics, and the resource of the draft is input to the machine learning model M. The machine learning model M to which the feature amount is input in this manner outputs the evaluation value of the policy flow of the draft.

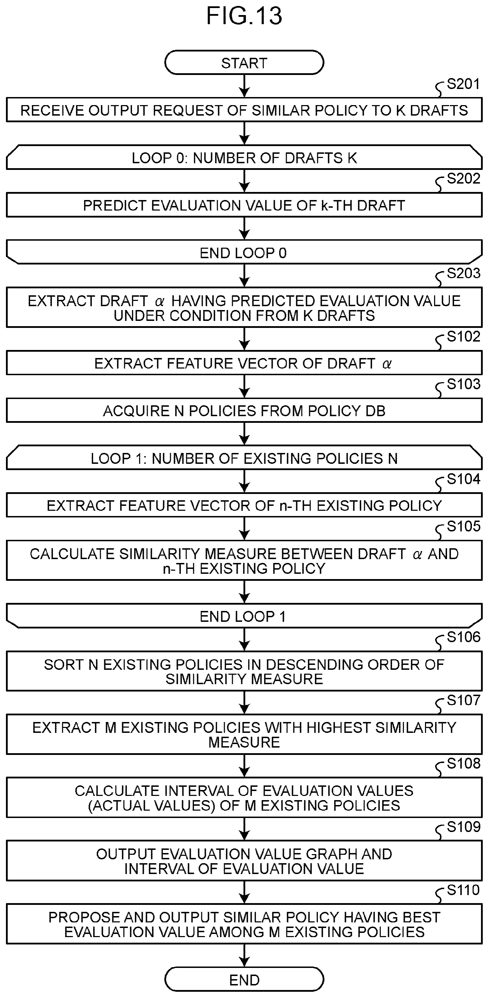

FIG. 13 is a flowchart illustrating a procedure of an output process of a similar policy. In the flowchart illustrated in FIG. 13, a step number at which a process similar to the one in the flowchart illustrated in FIG. 11 is executed is given a similar step number.

As illustrated in FIG. 13, when an output request of the similar policy of the K drafts is received by the reception unit 15A (step S201), the following process will be executed. That is, loop processing 0 of repeating the process of step S202 is executed by the number of times corresponding to the number of drafts K.

For example, the feature amount of at least one of the policy flow, the regional characteristics, and the resource of the K-th draft is input to the machine learning model M (step S202). As a result, the machine learning model M outputs the predicted evaluation value of the k-th draft.

By repeating such loop processing 0, a predicted evaluation value is obtained for each of the K drafts. Thereafter, the extraction unit 15C extracts, from the K drafts, a draft α having a predicted evaluation value under a predetermined condition, for example, the best value (step S203).

Then, the extraction unit 15C extracts the feature vector in which a feature amount obtained by quantifying the features of the policy flow of the draft α extracted in step S203 and the feature amount obtained by quantifying the features of the regional characteristics of the draft α are expressed in a vector format (step S102). Subsequently, the extraction unit 15C acquires N existing policies included in the policy DB 13A stored in the storage unit 13 (step S103).

Thereafter, loop processing 1 that repeats the processes of the following step S104 and the following step S105 is executed by the number of times corresponding to the number of existing policies N acquired in step S103.

That is, the extraction unit 15C extracts a feature vector in which a feature amount obtained by quantifying the features of the policy flow of the n-th existing policy and a feature amount obtained by quantifying the features of the regional characteristics of the n-th existing policy are expressed in a vector format (step S104).

Then, the first calculation unit 15D calculates similarity measure between the feature vector of the draft α extracted in step S102 and the feature vector of the n-th existing policy extracted in step S104 (step S105).

By repeating such loop processing 1, the similarity measure is calculated for each of the N existing policies.

Thereafter, the second calculation unit 15E sorts the N existing policies in descending order of the similarity measure calculated in step S105 (step S106). Subsequently, the second calculation unit 15E extracts M existing policies with the highest similarity measure (step S107).

Then, the second calculation unit 15E calculates an interval of evaluation values (actual values) of the M existing policies, for example, the maximum and minimum intervals or a confidence interval (step S108). Subsequently, the output unit 15F further plots an interval of the evaluation value calculated in step S108 on an evaluation value graph in which the evaluation values of the M similar policies corresponding to the evaluation axis are plotted for each evaluation axis, and causes the client terminal 30 to display the interval (step S109).

Furthermore, the output unit 15F causes the client terminal 30 to display, as a proposed policy, a similar policy whose evaluation value satisfies a predetermined condition among the M similar policies, for example, a similar policy corresponding to the best value or the highest specific number (step S110), and ends the process.

As described above, by executing the process from step S201 to step S203, the number of drafts to be compared with the existing policies can be narrowed down using the machine learning model for evaluation value prediction.

Note that, here, an example has been described in which a machine learning model for evaluation value prediction is used to narrow down the number of drafts to be compared with existing policies. A predicted evaluation value can be used by the second calculation unit 15E to determine a threshold of an evaluation value of a similar policy to be an extraction target of a confidence interval.

### <Application Example of Feature Amount>

In the first embodiment described above, an example has been described in which a feature vector including feature amounts of a policy flow and regional characteristics is generated, but the present invention is not limited thereto. For example, the feature vector may include, as the feature amount of the evaluation value, statistical values such as the average and variance of the BI score differences in the region, statistical values such as the average and variance of the hospital stay days in the region, and statistical values such as the average and variance of the hospital bed usage rate in the region. In addition, the feature vector may include, as the feature amount of the resource, statistical values such as the average and variance of medical workers in the region, and statistical values such as the average and variance of the number of medical resources (ambulances, helicopters, intensive care units (ICUs), magnetic resonance imaging (MRI), etc.) in the region.

### <Distribution and Integration>

In addition, each component of each device illustrated in the drawings may be physically configured other than as illustrated in the drawings. That is, a specific form of distribution and integration of each device is not limited to the illustrated form, and all or a part thereof can be functionally or physically distributed and integrated in arbitrary units according to various loads, usage conditions, and the like. For example, the reception unit 15A, the registration unit 15B, the extraction unit 15C, the first calculation unit 15D, the second calculation unit 15E, and the output unit 15F may be connected via a network as external devices of the server apparatus 10. In addition, the functions of the server apparatus 10 may be implemented by other devices including the reception unit 15A, the registration unit 15B, the extraction unit 15C, the first calculation unit 15D, the second calculation unit 15E, and the output unit 15F, being connected to a network and cooperating with one another.

### <Hardware Configuration>

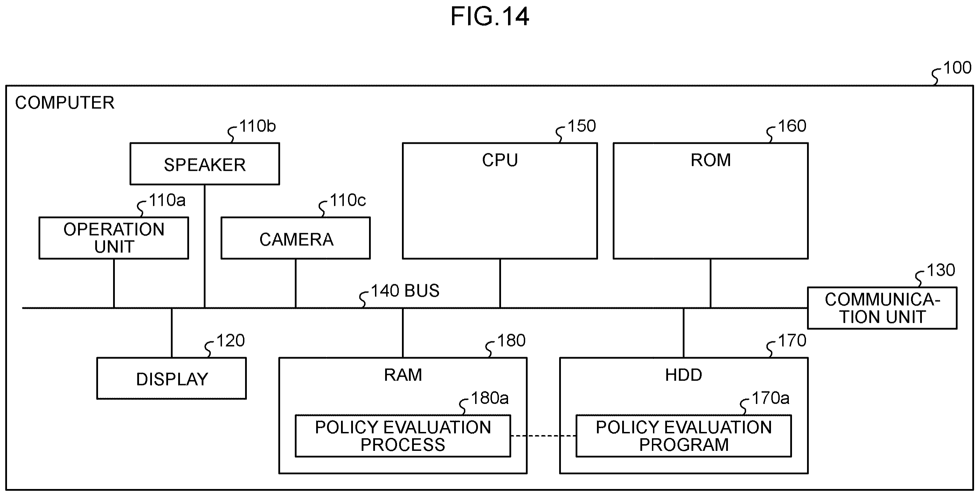

In addition, the various processes described in the above embodiment can be realized by executing a program prepared in advance on a computer such as a personal computer or a workstation. Therefore, an example of a computer that executes a policy evaluation program having the same functions as those of the first embodiment and the second embodiment will be described below with reference to FIG. 14.

FIG. 14 is a diagram illustrating an exemplary hardware configuration. As illustrated in FIG. 14, a computer 100 includes an operation unit 110a, a speaker 110b, a camera 110c, a display 120, and a communication unit 130. The computer 100 further includes a CPU 150, a ROM 160, an HDD 170, and a RAM 180. These units 110 to 180 are connected via a bus 140.

As illustrated in FIG. 14, the HDD 170 stores a policy evaluation program 170a that exhibits functions similar to those of the reception unit 15A, the registration unit 15B, the extraction unit 15C, the first calculation unit 15D, the second calculation unit 15E, and the output unit 15F described in the first embodiment described above. The policy evaluation program 170a may be integrated or separated similarly to the components of the reception unit 15A, the registration unit 15B, the extraction unit 15C, the first calculation unit 15D, the second calculation unit 15E, and the output unit 15F illustrated in FIG. 1. That is, the HDD 170 does not necessarily need to store all the data described in the first embodiment described above, and data used for the process may be stored in the HDD 170.

Under such an environment, the CPU 150 reads the policy evaluation program 170a from the HDD 170 and then deploys the program in the RAM 180. As a result, the policy evaluation program 170a functions as a policy evaluation process 180a as illustrated in FIG. 14. The policy evaluation process 180a deploys various data read from the HDD 170 into an area allocated to the policy evaluation process 180a in a storage area of the RAM 180, and executes various processes using the deployed various data. For example, as an example of the process executed by the policy evaluation process 180a, the process illustrated in FIGS. 11 and 13 and the like are included. Note that, in the CPU 150, not all the processing units described in the first embodiment described above need to be operated, and it is sufficient that a processing unit corresponding to a process to be executed is virtually realized.

The policy evaluation program 170a does not necessarily be stored in the HDD 170 or the ROM 160 from the beginning. For example, each program is stored in a "portable physical medium" such as a flexible disk, that is, a so-called FD, a CD-ROM, a DVD, a magneto-optical disk, or an IC card inserted into the computer 100. Then, the computer 100 may acquire and execute each program from these portable physical media. In addition, each program may be stored in another computer, a server apparatus, or the like connected to the computer 100 via a public line, the Internet, a LAN, a WAN, or the like, and the computer 100 may acquire and execute each program from the computer or the server apparatus.

### Reference Signs List

- 10: SERVER APPARATUS
- 11: COMMUNICATION CONTROL UNIT
- 13: STORAGE UNIT
- 13A: POLICY DB
- 15: CONTROL UNIT
- 15A: RECEPTION UNIT
- 15B: REGISTRATION UNIT
- 15C: EXTRACTION UNIT
- 15D: FIRST CALCULATION UNIT
- 15E: SECOND CALCULATION UNIT
- 15F: OUTPUT UNIT
- 30: CLIENT TERMINAL

## Claims

1. A policy evaluation method executed by a computer, the policy evaluation method comprising:
when acquiring a first policy that is a policy including a conditional branch and a node connected by a directed edge, referring to a storage unit that stores a plurality of second policies and an evaluation value of each of the plurality of second policies, the evaluation value indicating the number of targets to which a plurality of targets is assigned to a node by the policy, and calculating a similarity measure between the first policy and each of the plurality of second policies; and
outputting an evaluation value of a similar policy that is a policy including the conditional branch and the node having the similarity measure equal to or greater than a threshold among the plurality of second policies.

2. The policy evaluation method according to claim 1, wherein when the first policy and regional characteristics of the first policy are acquired, the calculating process includes referring to the storage unit that further stores regional characteristics of each of the plurality of second policies and calculating a similarity measure including a similarity between the regional characteristics of the first policy and the regional characteristics of each of the plurality of second policies.

3. The policy evaluation method according to claim 2, comprising extracting a first feature by using the first policy and regional characteristics of the first policy, wherein
the computer further executes a process of extracting a second feature by using the second policy and regional characteristics of the second policy, and
the calculating process includes calculating a similarity measure between the extracted first feature and the extracted second feature.

4. The policy evaluation method according to claim 2, wherein the regional characteristics are at least one of population distribution, demographics, traffic patterns, and facility deployment in a region.

5. The policy evaluation method according to claim 1 or 2, wherein the computer further executes a process of calculating a confidence interval of the evaluation values of the plurality of second policies using the distribution of the calculated similarity measure between the first policy and each of the plurality of second policies.

6. The policy evaluation method according to claim 1 or 2, wherein the computer further executes a process of displaying the evaluation value of the similar policy and the similarity measure in association with each other on a screen.

7. An information processing apparatus comprising a control unit configured to execute processes of:
when acquiring a first policy that is a policy including a conditional branch and a node connected by a directed edge, referring to a storage unit that stores a plurality of second policies and an evaluation value of each of the plurality of second policies, the evaluation value indicating the number of targets to which a plurality of targets is assigned to a node by the policy, and calculating a similarity measure between the first policy and each of the plurality of second policies; and
outputting an evaluation value of a similar policy that is a policy including the conditional branch and the node having the similarity measure equal to or greater than a threshold among the plurality of second policies.

8. The information processing apparatus according to claim 7, wherein when the first policy and regional characteristics of the first policy are acquired, the calculating process includes referring to the storage unit that further stores regional characteristics of each of the plurality of second policies and calculating a similarity measure including a similarity between the regional characteristics of the first policy and the regional characteristics of each of the plurality of second policies.

9. The information processing apparatus according to claim 8, comprising extracting a first feature by using the first policy and regional characteristics of the first policy, wherein
the control unit further executes a process of extracting a second feature by using the second policy and regional characteristics of the second policy, and
the calculating process includes calculating a similarity measure between the extracted first feature and the extracted second feature.

10. The information processing apparatus according to claim 8, wherein the regional characteristics are at least one of population distribution, demographics, traffic patterns, and facility deployment in a region.

11. The information processing apparatus according to claim 7 or 8, wherein the control unit further executes a process of calculating a confidence interval of the evaluation values of the plurality of second policies using the distribution of the calculated similarity measure between the first policy and each of the plurality of second policies.

12. The information processing apparatus according to claim 7 or 8, wherein the control unit further executes a process of displaying the evaluation value of the similar policy and the similarity measure in association with each other on a screen.

13. A policy evaluation program causing
a computer to execute processes of:
when acquiring a first policy that is a policy including a conditional branch and a node connected by a directed edge, referring to a storage unit that stores a plurality of second policies and an evaluation value of each of the plurality of second policies, the evaluation value indicating the number of targets to which a plurality of targets is assigned to a node by the policy, and calculating a similarity measure between the first policy and each of the plurality of second policies; and
outputting an evaluation value of a similar policy that is a policy including the conditional branch and the node having the similarity measure equal to or greater than a threshold among the plurality of second policies.

14. The policy evaluation program according to claim 13, wherein when the first policy and regional characteristics of the first policy are acquired, the calculating process includes referring to the storage unit that further stores regional characteristics of each of the plurality of second policies and calculating a similarity measure including a similarity between the regional characteristics of the first policy and the regional characteristics of each of the plurality of second policies.

15. The policy evaluation program according to claim 14, comprising extracting a first feature by using the first policy and regional characteristics of the first policy; and
causing the computer to further execute a process of extracting a second feature by using the second policy and regional characteristics of the second policy, wherein
the calculating process includes calculating a similarity measure between the extracted first feature and the extracted second feature.

16. The policy evaluation program according to claim 14, wherein the regional characteristics are at least one of population distribution, demographics, traffic patterns, and facility deployment in a region.

17. The policy evaluation program according to claim 13 or 14, causing the computer to further execute a process of calculating a confidence interval of the evaluation values of the plurality of second policies using the distribution of the calculated similarity measure between the first policy and each of the plurality of second policies.

18. The policy evaluation program according to claim 13 or 14, causing the computer to further execute a process of displaying the evaluation value of the similar policy and the similarity measure in association with each other on a screen.
